# EUROPEAN PATENT APPLICATION

(11) **EP 4 810 617 A1**
(43) Date of publication of application: **23.09.2026**
(21) Application number: 24890352.8
(22) Date of filing: 27.09.2024
(51) Int. Cl.: C12N 15/85, C12N 15/65, C12N 15/66, C12Q 1/68, A61K 39/00

(54) **RNA IN-VITRO CYCLIZATION AND ROLLING CIRCLE TRANSLATION METHOD BASED ON GROUP I INTRON, AND USE**

(30) Priority: 15.11.2023 CN 202311522587
(71) Applicant: Fudan University, Shanghai 200433 (CN)
(72) Inventor: QU, Liang, Shanghai 200433 (CN); YIN, Jie, Shanghai 200433 (CN); PAN, Qian, Shanghai 200433 (CN); WANG, Xinyue, Shanghai 200433 (CN)
(74) Representative: RGTH
(86) International application number: PCT/CN2024/121686
(87) International publication number: WO 2025/102994

(57) **Abstract**

The present disclosure discloses a method for RNA in vitro circularization and rolling circle translation based on a group I intron and use thereof. In the present disclosure, a specific non-complementary region of a group I intron is split to obtain a ribonucleic acid construct, and the preparation of "scarless" circular RNA is achieved through the ribozyme self-splicing principle. The method can also be extended to other group I introns. Through optimization of the splitting structure, efficient circularization can be achieved without homologous arms and without additional GTP catalysis. Meanwhile, the present disclosure also optimizes elements of the ribonucleic acid construct, further improving circularization efficiency. In particular, the present disclosure establishes a rolling circle translation (RCT) technology platform based on CITE sequences, and greatly improves expression levels of proteins or polypeptides. It has been verified that the circularization and rolling circle translation methods provided by the present disclosure exhibit potential in vaccines and CAR therapy, and are expected to be used in clinical treatment of various diseases.

## Description

### FIELD OF THE INVENTION

The present disclosure relates to the field of molecular biology and immunology, and in particular, to a method for RNA in vitro circularization and rolling circle translation based on a group I intron and use thereof.

### BACKGROUND

Circular RNA is a class of single-stranded RNA without a 5' cap and a 3' poly(A) tail and having a closed circular topological structure formed by covalent linkage. Due to the circular structure, circular RNA is protected from nuclease degradation and has relatively high stability. For a period of time after the discovery of circular RNA, circular RNA was considered to be a rare and nonfunctional abnormal splicing by-product. However, over the past decade, there has been a fundamental shift toward recognizing circular RNA as a ubiquitous and functionally important molecule in biology. This is largely attributed to the emergence of RNA-seq technology, which reveals that circular RNA is widespread and evolutionarily conserved in eukaryotes. Circular RNA has specific biological functions in RNA ligands, miRNA sponges, protein sponges, antisense circular molecules, innate immune activators, innate immune inhibitors, protein translation, and biomarkers.

In organisms, pre-mRNA is connected to form mRNA after introns are removed. This process occurs through the action of a spliceosome on the 5' splice site and the 3' splice site of the introns, thereby causing intron splicing and exon ligation to form mRNA, namely forward splicing. However, most circular RNA produced in organisms is not achieved by forward splicing, but rather by back splicing. Back splicing does not occur at both ends of an intron, but occurs at both ends of exons (i.e., the 3' end of the first intron and the 5' end of the second intron). Such splicing circularizes the exons to form circular RNA.

Due to the relatively high stability of circular RNA, the application of circular RNA in nucleic acid drugs has attracted attention, particularly in the in vitro preparation of circular RNA. Current methods mainly comprise: first, chemical synthesis through synthesis and ligation of special nucleotide derivatives; second, catalyzing terminal ligation of linear RNA through nucleic acid ligases; and third, connecting terminals of linear RNA based on the self-splicing property of intron ribozymes.

Nucleic acid drugs are another class of drugs extensively studied after small-molecule drugs and protein drugs, and circular RNA has achieved substantial development driven by linear RNA. Endogenous circular RNA can serve as a novel drug target or a biomarker for disease diagnosis, while artificially prepared circular RNA can target various targets and function within cells. Currently, circular RNA has achieved substantial breakthroughs in fields such as infectious vaccines, tumor vaccines, CAR-T, protein replacement therapy, and gene editing. Therefore, the preparation of circular RNA is crucial for the application of circular RNA.

Methods for preparing circular RNA based on the ribozyme self-splicing mechanism currently mainly comprise self-splicing methods based on group I introns and group II introns. Among them, group I intron sequences on the Anabaena tRNA Leu gene or the thymidylate synthase (td) gene of bacteriophage T4 are already employed for group I intron self-splicing, and both group I introns have been confirmed to efficiently promote RNA circularization. The main concept is to modify introns using a PIE system, generally comprising a 3' intron, an adjacent exon E2, a gene of interest, an adjacent exon E1, and a 5' intron. The circularization process of RNA is generally as follows: (1) a guanosine nucleophile attacks the E1 splice site and displaces the 3' terminus; and (2) the 3' terminus of the intermediate further attacks the E2 splice site, thereby forming circular RNA.

Recent reports disclose use of a Tetrahymena group I intron, where a trans-splicing ribozyme recognizes a target site (5'-NNNNNU-3') on a substrate RNA specifically complementary thereto through an internal guide sequence (IGS) (5'-GNNNNN-3'), thereby forming a P1 helical structure. The P1 and P10 helices respectively define the 5' splice site and the 3' splice site. By placing the target sequence recognized by the ribozyme at the 3' end of the gene of interest and placing the ribozyme sequence carrying the IGS at the 5' end of the gene of interest, circularization can be achieved without carrying AS, ABS, and P10 sequences (Lee, K.H., et al., Efficient circular RNA engineering by end-to-end self-targeting and splicing reaction using Tetrahymena group I intron ribozyme. Mol Ther Nucleic Acids, 2023. 33: p. 587-598).

Existing circularization technologies still have some unresolved problems. In preparation of circular RNA based on the ribozyme self-splicing mechanism, different RNA sequences result in enormous differences in the structure of the resulting circular RNA, leading to low circularization efficiency, high innate immunogenicity, and low expression. Splicing of group I introns from Ana and T4 introduces exogenous exon sequences, which may affect the function of circular RNA and may also lead to relatively high innate immunogenicity. The group I intron from Tetrahymena has relatively low circularization efficiency in the absence of homologous arms. Moreover, the translation efficiency of circular RNA also limits the application of this technology.

In view of this, there is a need for a circular RNA preparation and translation method capable of improving circularization efficiency, reducing exogenous sequences, lowering immunogenicity, and increasing expression levels, and thus the present disclosure is proposed.

### SUMMARY

To solve the above problems, the present disclosure innovatively splits the P9 non-complementary region of a group I intron and achieves the preparation of circular RNA based on the principle of ribozyme self-splicing. Through structural optimization, efficient circularization can be achieved without homologous arms and without additionally adding GTP, which is beneficial to industrial preparation. Meanwhile, the circularization method of the present disclosure introduces fewer exogenous sequences, thereby reducing innate immunogenicity and providing relatively high effectiveness and safety when applied to vaccines, in vivo CAR medicaments, and the like.

A first objective of the present disclosure is to provide a ribonucleic acid (RNA) construct for use in the preparation of circular RNA, and the construct comprises the following operable elements from the 5' end to the 3' end:
a 3' intron comprising a 3' splice site,
a GOI region comprising a gene of interest, and
5' intron comprising a 5' splice site,
wherein the 3' intron and the 5' intron are respectively two fragments obtained by splitting a group I intron, the 3' intron is a fragment comprising the 3' end of the group I intron, and the 5' intron is a fragment comprising the 5' end of the group I intron; the group I intron at least comprises, from the 5' end to the 3' end, 9 paired double-helical regions P1-P9, and the splitting site is located in a non-complementary region within the P9 region from the 5' end to the 3' end of the group I intron or in a region adjacent to the non-complementary region within the P9 region. That is, the 3' intron at least comprises a part of the P9 region at the 3' end of the group I intron, and the 5' intron at least comprises regions P1-P8 and another part of the P9 region at the 5' end of the group I intron.
the distance between the region adjacent to the non-complementary region within the P9 region and the 5' end or the 3' end of the non-complementary region of the P9 region is 0-40 nucleotides; and the group I intron is unmodified or has been subjected to optimization modification;
P9 is a collective term for subregions such as P9.0, P9, P9a, P9.1, P9.2, and P9.1a.

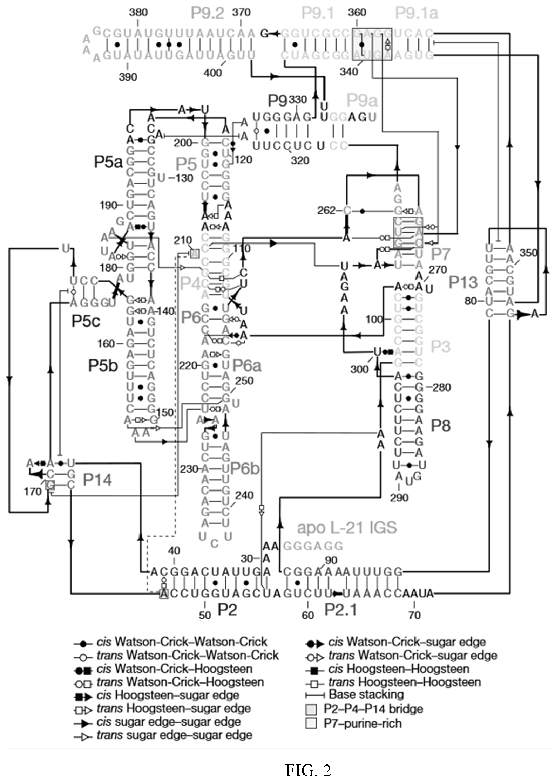

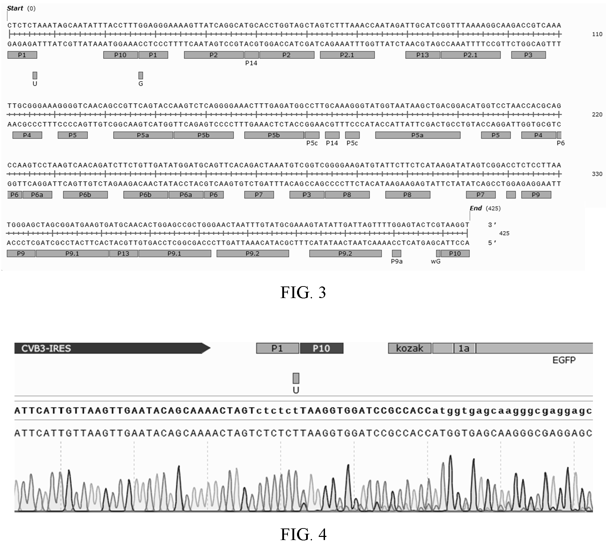

In the present disclosure:
(1) Definition of the splitting region:
   Through predictive analysis of homologous structures of group I introns, it is found that group I introns each possess 10 structurally conserved paired double-helical regions P1-P10 (from the 5' end to the 3' end), wherein each paired double-helical region may further comprise subregions, and the splitting site is located in a loop region of a stem-loop structure of the paired double-helical region or a subregion thereof, or in a region adjacent to the loop region.
(2) Definition of complementary regions and non-complementary regions:
   Taking the Tetrahymena group I intron of the present disclosure as an example, the specific sites of the complementary regions and non-complementary regions of P1-P10 are shown in FIGS. 2-3 (annotations of sequence sites of P1-P10 in FIGS. 2-3). These domain partitions are conserved and can be used to predict corresponding regions in other group I introns, thereby identifying complementary regions and non-complementary regions in other group I introns.
(3) The splice site:
   As well known to those skilled in the art, the 5' splice site and the 3' splice site are inherently present in the group I intron itself (as schematically shown in FIG. 3), and are described in detail below.

### Selection of the 5' splice site-internal guiding sequence (IGS):

Recognition of a substrate and selection of a cleavage site by a group I intron ribozyme are achieved through base pairing with the substrate. Among the two strands participating in formation of P1, the 3' strand is entirely derived from the intron and is referred to as an internal guiding sequence (IGS). The IGS pairs with the substrate sequence, and the formed P1 extends into the catalytic center of the ribozyme, thereby exposing the phosphorus atom at the 5' splice site on the substrate strand to the vicinity of a nucleophilic group, resulting in site-specific internal cleavage of the RNA molecule. The exon base preceding the 5' splice site is generally U, and the corresponding intron IGS base paired therewith is G. The resulting U:G pair is a characteristic base pair of the splice site. The U:G base pair at this position is conserved among all group I introns and plays an important role in splicing.

### Selection of the 3' splice site:

A short sequence proximal to the IGS can form a paired region referred to as P10 with the 3' exon proximal to the splice site. Although P10 contributes to the positioning of the 3' splice site, the selection of the 3' splice site does not depend on the formation of P10. The last nucleotide at the 3' end of the group I intron is generally G, referred to as ωG, and ωG has been demonstrated to be closely associated with the selection of the 3' splice site.

In summary, in the embodiments of the present disclosure, it is preferred that: the 3' intron comprises a 3' splice site and the target splice site comprises ωG, and the 5' intron comprises a 5' splice site and the target splice site forms a U:G pair with the IGS. Of course, other sites capable of achieving splicing can also be applied to the circularization method of the present disclosure.

Further, the group I intron at least comprises, from the 5' end to the 3' end, 10 paired double-helical regions P1-P10, and the splitting site is located in a non-complementary region within the P9 region from the 5' end to the 3' end of the group I intron or in a region adjacent to the non-complementary region within the P9 region. That is, the 3' intron at least comprises a part of the P9 region and the P10 region at the 3' end of the group I intron, and the 5' intron at least comprises regions P1-P8 and a part of the P9 region at the 5' end of the group I intron. That is, the present disclosure attempts to remove the P10 region of the group I intron and finds that the above RNA construct still achieves efficient circularization.

Further, it is found in the present disclosure that, relative to other splitting regions, the ribonucleic acid construct formed by the 5' intron and the 3' intron obtained by splitting in the P9.2 non-complementary region of the P9 region or in a region adjacent to the P9.2 non-complementary region exhibits the highest circularization efficiency during the preparation of circular RNA.

Further, the group I intron is derived from, but is not limited to, *Tetrahymena*, *Anabaena*, bacteriophage T4, and the like.

Further, the modification comprises modification of the complementary regions of P1-P10 (or P1-P9), and the modification comprises one or more of addition, deletion, and mutation.

Further, the modification comprises modification of the non-complementary region of P1-P10 (or P1-P9), and the modification comprises one or more of addition, deletion, and mutation.

Further, the ribonucleic acid construct further comprises an optimized sequence for promoting circularization and expression. Preferably, the optimized sequence is selected from one or more of a homologous arm, a spacer sequence, and an untranslated region (UTR) sequence.

Preferably, at least one of the following is comprised:
(1) the homologous arm comprising an external homologous arm and/or an internal homologous arm;
(2) the spacer sequence comprising a polyA sequence or a polyAC sequence.

Further, the homologous arm is located on an extension arm of the complementary regions of P1-P10 (or P1-P9) of the group I intron before splitting, or is located at the 5' end or the 3' end of the 3' intron or the 5' intron after splitting. That is, when the homologous arm is an external homologous arm, in the RNA construct, the external homologous arm is located at the 5' end of the 3' intron and/or at the 3' end of the 5' intron; when the homologous arm is an internal homologous arm, in the RNA construct, the internal homologous arm is located at the 3' end of the 3' intron and/or at the 5' end of the 5' intron.

Preferably, the length of the homologous arm is 10-1000 bp, more preferably 10-500 bp, and most preferably 10-200 bp.

Further, the spacer sequence is located upstream of the IRES sequence (or CITE sequence) and/or downstream of the GOI; the 5'UTR sequence is located upstream of the IRES sequence (or CITE sequence), and the 3'UTR sequence is located downstream of the GOI sequence.

Further, in addition to the coding sequence of the gene of interest, the GOI region further comprises a sequence for initiating translation. The sequence for initiating translation comprises, but is not limited to, an internal ribosome entry site (IRES) sequence, an m6A motif, a cap-independent translation enhancer (CITE) sequence, a kozak sequence, and the like.

The IRES sequence is a nucleotide sequence having cap structure-independent translation initiation ability and is capable of recruiting ribosomes to translate mRNA with the assistance of trans-acting factors. The kozak sequence is an important sequence for initiation of translation in eukaryotes and is present in the translation initiation region of mRNA. The kozak sequence directs translation elements to read mRNA and determine the correct initiation site, thereby ensuring correct protein synthesis. However, commonly used internal ribosome entry site (IRES) translation element sequences are relatively long and comprise a plurality of stop codons, and therefore rolling circle translation cannot be achieved. Therefore, the present disclosure obtains a cap-independent translation enhancer (CITE) element capable of performing rolling circle translation and compatible with base modification through screening of novel cap-independent translation initiation elements. Unlike IRES, which directly recruits ribosomes to mediate translation, CITE mediates translation through binding to translation initiation factors (eIFs). On this basis, the present disclosure develops a variety of novel RNA technologies capable of performing rolling circle translation, significantly improving the expression level and half-life of proteins. Herein, the present disclosure develops a novel self-splicing ribozyme circularization scheme capable of achieving "scarless" circularization, thereby minimizing the introduction of exogenous sequences and reducing the influence of redundant sequences on polypeptide function.

Further, at least one of the following is comprised:
(a) the IRES sequence being derived from Simian V4, CVB3, CVB5, CVB1, HRV-A89, HRV-B3, Salivirus A GUT, EV107, CVA3, HRV-B37, EchoV11, HRV-C54, HRV-B4, EV-D94, HRV-B93, EV-J, SwineVesicular, CVA20, PV1, HRV-A1, Salivirus A SZ1, Salivirus FHB, EMCV-cf, HCV, Aichivirus, CrPV, Covid 19, Crohivirus B, EchoV-E11, Sapevirus, Phopivirus, or the like; and
(b) the CITE sequence being derived from a 5'UTR or a 3'UTR of PMV, CarMV-PTE, CbMV, CCFV, CIRV, GaMV, HCRSV, HnRSV, MNeSV, MNSV, PLPV, PEMV2, PFBV, PSNV, RCNMV1, SCV, TBSV, TCV, TNV, BBV, BYDV, STNV, SCV, PEMV2, TPAV, STNV, or other viruses; or being derived from a 5'UTR or a 3'UTR of HIF-1α, FGF9, p53A, p53B, or other genes in the Homo sapiens genome.

Further, the coding sequence of the gene of interest in the ribonucleic acid construct comprises, but is not limited to, a viral antigen sequence, a tumor antigen sequence, a monoclonal antibody sequence, a bispecific antibody sequence, a CAR sequence, a cytokine sequence, a coagulation factor sequence, a growth factor sequence, a gene-editing enzyme sequence, a fluorescent protein sequence, a luciferase sequence, and the like.

Further, the ribonucleic acid construct further comprises essential elements required for expression, such as a promoter. Of course, other elements may also be added according to actual requirements.

A second objective of the present disclosure is to provide a circular RNA obtained by circularization of the above ribonucleic acid construct.

A third objective of the present disclosure is to provide a preparation method for a circular RNA, wherein the circular RNA is obtained by circularization of the above ribonucleic acid construct. Specifically, circular RNA can be efficiently prepared in the absence of a homologous arm sequence and without addition of GTP, and the resulting circular RNA contains fewer exogenous sequences, exhibits low immunogenicity, and achieves high expression in cells.

Further, a plasmid containing the gene of interest is linearized, and circular RNA is obtained by using in vitro transcription (IVT) technique, DNaseI treatment, and RNA purification. The circular RNA is further purified and enriched through different treatments, comprising RNase R, HPLC, and CIP, thereby preparing high-purity circular RNA.

Further, the in vitro transcription is performed for at least 0.5 h, preferably 4-16 h.

Further, the purified circular RNA is subjected to RNase R treatment, and electrophoresis is performed to observe whether circularization is successful. The purified circular RNA is subjected to reverse transcription-PCR (RT-PCR), and the target band is sequenced to confirm successful preparation of the circular RNA and correctness of the ligation site.

Further, the purified circular RNA is transfected into 293T cells, and after 24 hours, its high expression is detected through fluorescence microscopy and flow cytometry.

Further, modified nucleotides are added during the preparation process at an addition ratio of 0%-100%.

A fourth objective of the present disclosure is to provide a recombinant cell comprising the ribonucleic acid construct or the circular RNA. The host cell may be any eukaryotic cell or prokaryotic cell and can be applied in the pharmaceutical field (preferably a human-derived cell).

A fifth objective of the present disclosure is to provide a method for preparing a protein or polypeptide using circular RNA, wherein the ribonucleic acid construct is prepared into circular RNA, the circular RNA is introduced into a host cell for protein expression, and the gene of interest encodes the protein or polypeptide.

A sixth objective of the present disclosure is to protect use of the ribonucleic acid construct or the circular RNA described above in the preparation of a diagnostic product, a prophylactic product, or a therapeutic product.

A seventh objective of the present disclosure is to prepare a medicament, wherein the medicament comprises the ribonucleic acid construct or the circular RNA, or a variant based thereon (e.g., a recombinant cell or an expression vector carrying the sequence).

Further, the medicament is a vaccine, and specifically, the vaccine comprises the ribonucleic acid construct or the circular RNA, wherein the gene of interest is an antigen-encoding sequence. For example, when the antigen is a B-cell epitope or a T-cell epitope, neutralizing antibodies and T-cell immune responses are generated after injection into a body, thereby achieving resistance against infectious diseases and tumors.

Further, the vaccine further comprises a liposome delivery carrier.

Further, the medicament is a CAR immunotherapeutic medicament comprising a circular chimeric antigen receptor (CAR) molecule, that is, the gene of interest in the above circular RNA is a CAR-encoding sequence. Specifically, a circular RNA molecule expressing a chimeric antigen receptor is injected into a body and targeted to a target site in vivo, so as to engineer in vivo immune cells in the body, thereby achieving immunotherapy and resisting tumors.

Further, the CAR immunotherapeutic medicament further comprises a delivery carrier having a targeting function. Specifically, the ribonucleic acid construct is prepared into a circular RNA medicament, which enters a body through the delivery carrier having a targeting function to generate in vivo CAR, wherein the targeted immune cells comprise T cells, macrophages, natural killer cells, neutrophils, γδT cells, and the like, thereby generating engineered immune cells in vivo, characterized in that an in vivo immunotherapeutic medicament against tumors is prepared.

Further, the medicament can be used for the treatment of various diseases, particularly diseases that can be ameliorated through gene therapy, comprising but not limited to tumors (solid tumors or non-solid tumors), autoimmune diseases, and the like. For example, the medicament is an immunotherapeutic medicament for autoimmune encephalomyelitis (EAE), comprising a circular antigen molecule, that is, the gene of interest in the above circular RNA is an MOG35-55 short peptide-encoding sequence. Specifically, a circular RNA molecule expressing an antigen receptor is injected into a body and targeted to a target site in vivo, so as to engineer in vivo immune cells in the body, thereby achieving immunotherapy.

In the embodiments of the present disclosure, in terms of vaccine application, short-sequence circRNA is synthesized using an optimized "scarless" circularization scheme, and the immune activation effect in vivo of a rolling circle translation circular RNA vaccine encoding the OVA antigen peptide is analyzed through delivery of the model antigen OVA antigen peptide by a lipid nanoparticle (LNP) delivery mode. Compared with linear mRNA, the circular RNA vaccine exhibits better antitumor effects and T-cell immune responses. The present disclosure provides a novel method for preparing a circular RNA vaccine against infectious diseases and tumors capable of generating efficient T-cell immune responses. In terms of chimeric antigen receptor (CAR) therapy, the present disclosure provides a method for engineering immune cells in vivo (comprising T cells, NK cells, macrophages, and the like) based on circular RNA "off-the-shelf" *in vivo* CAR, and antitumor *in vivo* CAR immunotherapeutic medicaments can be developed therefrom. In terms of immunotherapy, the present disclosure establishes an autoimmune encephalomyelitis (EAE) mouse model, prepares a circular RNA capable of performing rolling circle translation using the circularization scheme of the present disclosure, and uses the same for the treatment of autoimmune diseases.

The beneficial effects of the present disclosure are as follows:
1) The present disclosure uses the Tetrahymena group I intron and achieves a relatively high circularization rate without addition of GTP. In addition, efficient circularization can also be achieved for large-molecule RNA, thereby facilitating scale-up industrial production.
2) Compared with circularization based on other group I introns, the present disclosure introduces fewer exogenous sequences, thereby resulting in lower innate immunogenicity, reduced toxic and side effects, and increased expression level.
3) Compared with circularization based on other group I introns, the present disclosure achieves efficient preparation of circular RNA without addition of homologous arms.
4) The present disclosure achieves RNA circularization based on the self-splicing principle through splitting of the non-complementary region in the Tetrahymena group I intron. The method can also be extended to other group I introns to achieve effective circularization.
5) The present disclosure achieves a novel circularization scheme for group I introns, which has excellent application in nucleic acid drugs (such as vaccines and in vivo CAR therapeutics), exhibits relatively high stability, and does not integrate into the genome, thereby reducing the risk of carcinogenesis.

### BRIEF DESCRIPTION OF THE DRAWINGS

FIG. 1 is a schematic diagram of the design of the circular RNA construct of the present disclosure.
FIG. 2 shows the reconstructed secondary structure of the Tetrahymena group I intron by cryo-electron microscopy, where the thick lines with arrows indicate backbone continuity;
lines with different symbols indicate different base pairing and stacking; and dashed lines indicate hydrogen bonds present in the P2-P4-P14 bridge.
FIG. 3 shows the gene sequence of the Tetrahymena group I intron and partition annotations of P1-P10.
FIG. 4 shows sequencing results of circular RNA ligation site. Circular RNA-EGFP is reverse transcribed into cDNA and subjected to subsequent PCR amplification using specific primers. Sanger sequencing analysis of the PCR product shows precise ligation mediated by the PICC system.
FIG. 5 shows fluorescence microscopy observation results of GFP expression from circular RNA generated by different circularization schemes 24 hours after transfection of circular RNA into HEK293T cells.
FIG. 6 shows quantitative analysis results, by flow cytometry, of differences in GFP expression levels of circular RNA generated by PICC and STS-P1 circularization schemes 24 hours after transfection of circular RNA into HEK293T cells.

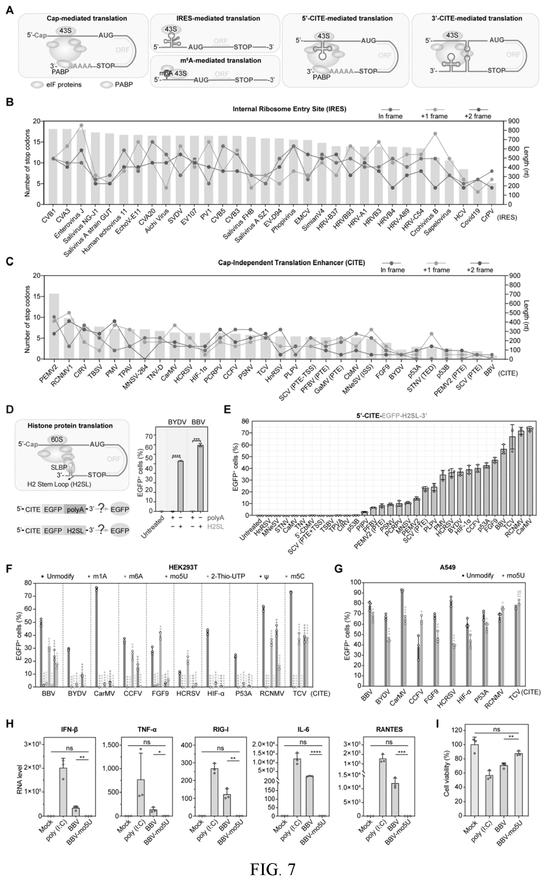

FIG. 7 shows results of screening of cap-independent translation enhancer (CITE) sequences and immunogenicity detection after modification. (A), multiple translation initiation mechanisms are summarized, comprising conventional cap-dependent and cap-independent strategies, and the latter is further subdivided into IRES-, m6A-, and CITE-mediated methods; (B-C), characteristics of 32 IRES and 30 CITE elements are analyzed, comprising sequence lengths thereof and the number of stop codons in the in-frame (green), +1 frame (yellow), and +2 frame (blue); (D), HEK293T cells are transfected with equal amounts of RNA expressing various engineered CITE structures, where the left panel shows percentages of EGFP-positive cells and the right panel shows different structures; (E) HEK293T cells are transfected with equal amounts of RNA to evaluate translation ability of 31 CITEs by detecting percentages of EGFP-positive cells; (F) HEK293T cells are transfected with equal amounts of RNA to evaluate compatibility with different base modifications, comprising m1A, m6A, mo5U, 2-Thio-UTP, ψ, and m5C, and compatibility between different CITEs and these base modifications is detected; (G) in A549 cells, equal amounts of RNA encoding EGFP are used for transfection to evaluate compatibility between multiple CITEs and mo5U-modified bases; (H) 6 hours after transfection, relative expression levels of innate immune genes (IFN-β, TNF-α, RIG-I, IL-6, and RANTES) in A549 cells are determined by qRT-PCR, where relative fold changes are normalized using cells in the mock group; and (I), 24 hours after transfection, cell viability of A549 cells transfected with equal amounts of mo5U-modified or unmodified RNA is detected. Data are expressed as mean ± standard deviation (S.D.). Comparison is performed using a two-sided unpaired Student's t-test. The following symbols represent significance levels: *p < 0.05; **p < 0.01; ***p < 0.001; ****p < 0.0001; ns represents not significant.

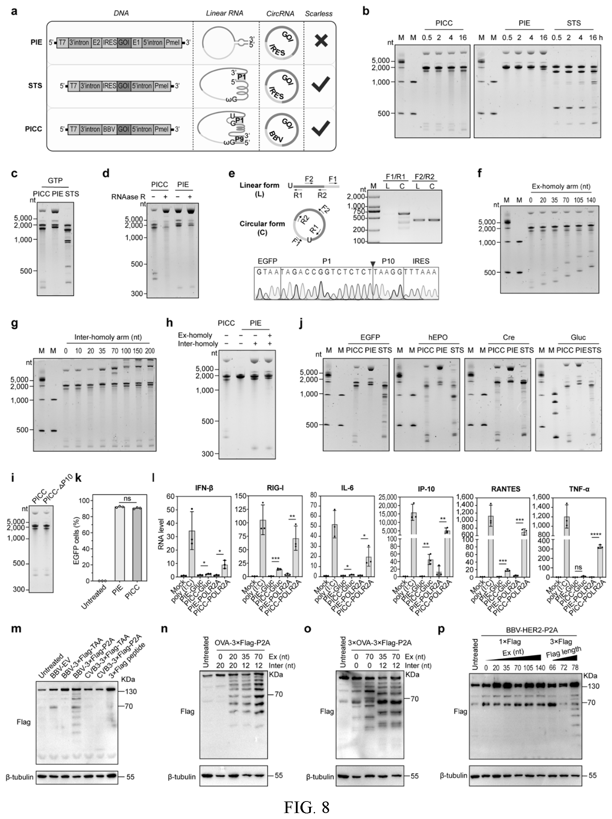

FIG. 8 shows comparison results between the circularization scheme used in the present disclosure and other circularization schemes, as well as verification results of rolling circle translation of the circular RNA constructed based on the present disclosure. (A), the schematic diagram shows an in vitro circularization system for circular RNA. The upper panel shows a PIE system, in which target RNA is generated from a DNA template through in vitro transcription (IVT) and circularized through autocatalytic splicing of a rearranged *Anabaena* pre-tRNA group I intron. The middle panel shows an STS system, in which target RNA is generated through IVT and circularized through a P1-splitting mode of autocatalytic splicing of a *Tetrahymena* group I intron. The lower panel shows a PICC system, in which target RNA is generated through IVT and circularized through a P9-splitting mode of autocatalytic splicing of a *Tetrahymena* group I intron. (B), circularization efficiencies of the three in vitro circularization systems for generating circRNA^{EGFP} at different IVT reaction times are determined by urea-polyacrylamide gel electrophoresis (Urea-PAGE) under denaturing conditions. (C), circularization efficiencies of the three in vitro circularization systems for generating circRNA^{EGFP} after GTP treatment are determined by urea-polyacrylamide gel electrophoresis (Urea-PAGE) under denaturing conditions. (D), circularization efficiencies of the PICC and PIE systems for generating circRNA^{EGFP} are evaluated after RNase R treatment for 30 minutes by urea-polyacrylamide gel electrophoresis (Urea-PAGE) under denaturing conditions. (E), precise ligation mediated by the PICC system. Upper panel: the schematic diagram shows a process in which linear RNA precursors and circRNA^{EGFP} are reverse transcribed into cDNA and subsequently subjected to PCR amplification using specific primers (left), and agarose gel electrophoresis analysis results of PCR products (right). Lower panel: sequencing analysis results of PCR products. (F), circularization efficiencies of the PICC system for generating circRNA^{EGFP} through addition of external homologous arms of different lengths are determined by urea-polyacrylamide gel electrophoresis (Urea-PAGE) under denaturing conditions. (G), circularization efficiencies of the PICC system for generating circRNA^{EGFP} through addition of internal homologous arms of different lengths are determined by urea-polyacrylamide gel electrophoresis (Urea-PAGE) under denaturing conditions. (H), circularization efficiencies of the PICC and PIE systems for generating circRNA^{EGFP} through addition of internal or/and external homologous arms are determined by urea-polyacrylamide gel electrophoresis (Urea-PAGE) under denaturing conditions. (I), circularization efficiencies of the PICC system are evaluated by urea-polyacrylamide gel electrophoresis (Urea-PAGE) under denaturing conditions in the presence or absence of P10 (a structural component of the group I intron ribozyme) (lane 1: PICC-RNA construct; lane 2: PICC-RNA construct without P10 structure). (J), circularization efficiencies of precursor RNA comprising a CVB3 IRES and inserted sequences (comprising coding regions of Gaussia luciferase (GLuc), human erythropoietin (hEpo), EGFP, or Cre) are evaluated by urea-polyacrylamide gel electrophoresis (Urea-PAGE) under denaturing conditions. (K), percentages of EGFP-positive cells in HEK293T cells transfected with EGFP-encoding circular RNA generated by the PICC and PIE systems are determined by flow cytometry. (L), within 6 hours after transfection with circRNA-Gluc and circRNA-POLR2A generated by the PICC and PIE systems, relative expression levels of immunogenicity-related genes (IFN-β, RIG-I, IL-6, IP-10, RANTES, and TNF-α) in A549 cells are determined by qRT-PCR. Relative fold changes are normalized using cells in the mock group. (M), expression of Flag protein in HEK293T cells is detected by western blot after transfection with linear mRNA or circular RNA generated by the PICC system mediated by BBV or CVB3 sequences and comprising a stop codon or a P2A element. (N and O), expression of 1×OVA and 3×OVA proteins encoded by PICC-circRNA-BBV with added internal or/and external homologous arms in HEK293T cells is detected by western blot. Screening results of circular RNA-OVA-3×Flag-P2A are shown in (N), and screening results of circular RNA-3×OVA-3×Flag-P2A are shown in (O). (P), expression of HER2 protein in HEK293T cells from circular RNA generated by the PICC system with addition of different lengths of 3×Flag sequences or internal or/and external homologous arm sequences is detected by western blot. Data are expressed as mean ± standard deviation (S.D.). The following symbols represent significance levels: *p < 0.05; **p < 0.01; ***p < 0.001; ****p < 0.0001; ns represents not significant.

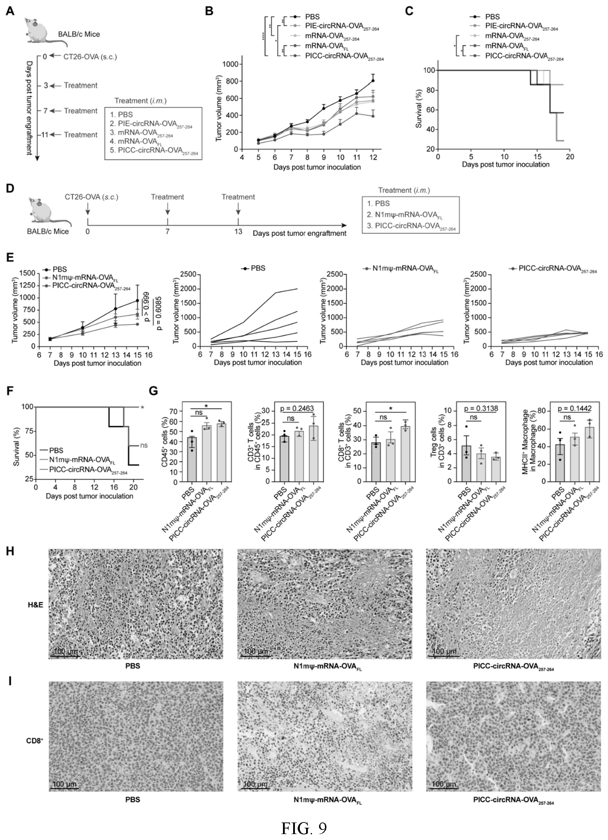

FIG. 9 shows in vivo verification results in mice of rolling circle translation of the OVA antigen by the circular RNA constructed in the present disclosure. (A), OVA+ CT26 tumor-bearing mice receive treatments comprising PBS and a series of RNA vaccines, where the vaccines comprise mRNA encoding OVA257-264, mRNA encoding full-length OVA, circular RNA encoding OVA257-264 generated by the PIE system, and circular RNA encoding OVA257-264 generated by the PICC system. (B), tumor growth curves of tumor-bearing mice. Tumor volume is monitored daily 5 days after tumor inoculation. (C), statistics of survival rate of tumor-bearing mice. Compared to the group with expression of full-length OVA, mice receiving PICC-circRNA-OVA₂₅₇₋₂₆₄ treatment (expressing OVA short peptide through rolling circle translation) exhibit better survival rates. (D), OVA⁺ CT26 tumor-bearing mice receive a series of interventions comprising PBS, modified mRNA encoding full-length OVA, and circular RNA encoding OVA₂₅₇₋₂₆₄ generated by the PICC system. (E), tumor growth curves of tumor-inoculated mice. Tumor volume is monitored after the first administration. (F), statistics of survival rate of tumor-bearing mice. Compared with the N1mΨ-modified mRNA group, mice receiving PICC-circRNA-OVA₂₅₇₋₂₆₄ treatment exhibit better survival rates. (G), infiltration of CD45⁺ immune cells in tumors, proportions of CD8⁺ T cells among tumor-infiltrating CD45⁺ cells, proportions of Treg cells among tumor-infiltrating CD45⁺ cells, and proportions of MHCII⁺ macrophages among tumor-associated macrophages are detected by flow cytometry. (H), H&E staining is performed on tumor tissue sections from CT26-OVA tumor-bearing mice receiving PBS, N1mΨ-mRNA-OVAFL, or PICC-circRNA-OVA₂₅₇₋₂₆₄ treatment. (I), IHC staining of CD8⁺ cells is performed on tumor tissue sections from CT26-HER2 tumor-bearing mice receiving PBS, N1mΨ-mRNA-OVA_{FL}, or PICC-circRNA-OVA₂₅₇₋₂₆₄ treatment. Data are expressed as mean ± standard error (S.E.M.). In (B and E), tumor growth curves are analyzed using two-way ANOVA. In (C and F), survival curves are analyzed using Kaplan-Meier survival analysis. In (G), comparison is performed using a two-sided Student's t-test. The following symbols represent significance levels: *p < 0.05; **p < 0.01; ***p < 0.001; ****p < 0.0001; ns represents no significant difference.

### DETAILED DESCRIPTION

The present disclosure will be further described below with reference to the drawings and specific examples, such that those skilled in the art can better understand and implement the present disclosure. However, the examples given should not be construed as limiting the present disclosure.

The sequences involved in the following examples are as follows:
(1) Sequences required for constructing PICC-circRNA structure:
   T7 promoter (SEQ ID NO. 1):
   TAATACGACTCACTATAGG
   3' intron (SEQ ID NO. 2):
   AAGTATATTGATTAGTTTTGGAGTACTCGTAAGGT
   Kozak sequence (SEQ ID NO. 3):
   GCCACC
   CVB3 IRES sequence (SEQ ID NO. 4): 
TTAAAACAGCCTGTGGGTTGATCCCACCCACAGGCCCATTGGGCGCTAGCACTCTGGTATCACGGTAC
CTTTGTGCGCCTGTTTTATACCCCCTCCCCCAACTGTAACTTAGAAGTAACACACACCGATCAACAGTC
AGCGTGGCACACCAGCCACGTTTTGATCAAGCACTTCTGTTACCCCGGACTGAGTATCAATAGACTGC
TCACGCGGTTGAAGGAGAAAGCGTTCGTTATCCGGCCAACTACTTCGAAAAACCTAGTAACACCGTG
GAAGTTGCAGAGTGTTTCGCTCAGCACTACCCCAGTGTAGATCAGGTCGATGAGTCACCGCATTCCCC
ACGGGCGACCGTGGCGGTGGCTGCGTTGGCGGCCTGCCCATGGGGAAACCCATGGGACGCTCTAAT
ACAGACATGGTGCGAAGAGTCTATTGAGCTAGTTGGTAGTCCTCCGGCCCCTGAATGCGGCTAATCCT
AACTGCGGAGCACACACCCTCAAGCCAGAGGGCAGTGTGTCGTAACGGGCAACTCTGCAGCGGAAC
CGACTACTTTGGGTGTCCGTGTTTCATTTTATTCCTATACTGGCTGCTTATGGTGACAATTGAGAGATC
GTTACCATATAGCTATTGGATTGGCCATCCGGTGACTAATAGAGCTATTATATATCCCTTTGTTGGGTT
TATACCACTTAGCTTGAAAGAGGTTAAAACATTACAATTCATTGTTAAGTTGAATACAGCAAA
   Nucleotide sequence of GFP (SEQ ID NO. 5): 
ATGGTGAGCAAGGGCGAGGAGCTGTTCACCGGGGTGGTGCCCATCCTGGTCGAGCTGGACGGCGAC
GTAAACGGCCACAAGTTCAGCGTGTCCGGCGAGGGCGAGGGCGATGCCACCTACGGCAAGCTGACC
CTGAAGTTCATCTGCACCACCGGCAAGCTGCCCGTGCCCTGGCCCACCCTCGTGACCACCCTGACCTA
CGGCGTGCAGTGCTTCAGCCGCTACCCCGACCACATGAAGCAGCACGACTTCTTCAAGTCCGCCATGC
CCGAAGGCTACGTCCAGGAGCGCACCATCTTCTTCAAGGACGACGGCAACTACAAGACCCGCGCCGA
GGTGAAGTTCGAGGGCGACACCCTGGTGAACCGCATCGAGCTGAAGGGCATCGACTTCAAGGAGGA
CGGCAACATCCTGGGGCACAAGCTGGAGTACAACTACAACAGCCACAACGTCTATATCATGGCCGAC
AAGCAGAAGAACGGCATCAAGGTGAACTTCAAGATCCGCCACAACATCGAGGACGGCAGCGTGCAG
CTCGCCGACCACTACCAGCAGAACACCCCCATCGGCGACGGCCCCGTGCTGCTGCCCGACAACCACTA
CCTGAGCACCCAGTCCGCCCTGAGCAAAGACCCCAACGAGAAGCGCGATCACATGGTCCTGCTGGAG
TTCGTGACCGCCGCCGGGATCACTCTCGGCATGGACGAGCTGTACAAGTAA
   5' intron (SEQ ID NO. 6): 
CTCTCTAAATAGCAATATTTACCTTTGGAGGGAAAAGTTATCAGGCATGCACCTGGTAGCTAGTCTTT
AAACCAATAGATTGCATCGGTTTAAAAGGCAAGACCGTCAAATTGCGGGAAAGGGGTCAACAGCCGT
TCAGTACCAAGTCTCAGGGGAAACTTTGAGATGGCCTTGCAAAGGGTATGGTAATAAGCTGACGGAC
ATGGTCCTAACCACGCAGCCAAGTCCTAAGTCAACAGATCTTCTGTTGATATGGATGCAGTTCACAGA
CTAAATGTCGGTCGGGGAAGATGTATTCTTCTCATAAGATATAGTCGGACCTCTCCTTAATGGGAGCT
AGCGGATGAAGTGATGCAACACTGGAGCCGCTGGGAACTAATTTGTATGCGA
   Tetrahymena group I intron (SEQ ID NO. 7): 
CTCTCTA1AATAGCAATATTTACCTTTGGAGGGAAAAGTTATCAGGCATGCACCTGGTAGCTAGTCTTT
AAACCAATAGATTGCATCGGTTTAAAAGGCAAGACCGTCAAATTGCGGGAAAGGGGTCAACAGCCGT
TCAGTACCAAGTCTCAGGGGAAACTTTGAGATGGCCTTGCAAAGGGTATGGTAATAAGCTGACGGAC
ATGGTCCTAACCACGCAGCCAAGTCCTAAGTCAACAGATCTTCTGTTGATATGGATGCAGTTCACAGA
CTAAATGTCGGTCGGGGAAGATGTATTCTTCTCATAAGATATAGTCGGACCTCTCCTTAATGGGAGCT
AGCGGATGAAGTGATGCAACACTGGAGCCGCTGGGAACTAATTTGTATGCGA384A385AGTATATTGA
TTAGTTTTGGAGTACTCGTAAGGT
   Tetrahymena group I intron without P10 (SEQ ID NO. 8): 
CTCTCTA1AATAGCAATATTTACCTTTGGAGGGAAAAGTTATCAGGCATGCACCTGGTAGCTAGTCTTT
AAACCAATAGATTGCATCGGTTTAAAAGGCAAGACCGTCAAATTGCGGGAAAGGGGTCAACAGCCGT
TCAGTACCAAGTCTCAGGGGAAACTTTGAGATGGCCTTGCAAAGGGTATGGTAATAAGCTGACGGAC
ATGGTCCTAACCACGCAGCCAAGTCCTAAGTCAACAGATCTTCTGTTGATATGGATGCAGTTCACAGA
CTAAATGTCGGTCGGGGAAGATGTATTCTTCTCATAAGATATAGTCGGACCTCTCCTTAATGGGAGCT
AGCGGATGAAGTGATGCAACACTGGAGCCGCTGGGAACTAATTTGTATGCGA384A385AGTATATTGA
TTAGTTTTGGAGTACTCG
(2) CITE sequences required for optimizing PICC-circRNA structure:
   BBV (SEQ ID NO. 9):
      UGUUUAGGUUCGCUUUUCUGUUUUAUUUGUUUCGAAAAC
   BYDV (SEQ ID NO. 10): 
GUGAAGAUUGACCAUCUCACAAAAGCUGUUACGUGCUUGUAACACACUACUCACUCGUUUUGU
AUUCGAGAAGUAGUUGCAACAACGGUUCCCUCAUUGCCUGACAAGCUGAGGGCCACCCUUCUAU
CCCCACCGCCAUC
   CarMV (SEQ ID NO. 11): 
AUGCAAGAGAAAAGUUUGGUAGACACACACGGCGGGACGUGUUGCUAGAGCUGCCACUCUGCC
CGAGAUGGAUCGAUAUUAACCAUCUUUGGCAACAGCGUGUGUGAUCGACCAAACCUAGUAAUU
GCGUAGCAUCCGACUGUAACCUGUUCUCUGCUGAAUGCGAGGGUGGCCUCGUGAUCCCAAACCA
UAAAAGAAACCAACUUGUUGGUGGAAGGGCUUGGAAGUAGGAUCAACCACUGUUUAUUAAUUA
GGGGUGAGAGUAUUCACUGUACUCUUUCCCCGCCC
   CCFV (SEQ ID NO. 12): 
AGUAAGUAGCCUAACCUGCUGCAGCAGGAAGUAUUCUAGGUAGUAUAGUAACUCUUUCUUCAA
CUUCUUACUUAGAAAAACACAAAAAUACCCCCAGCUCGCUUUUGUUUUGAGCUGUGUUAGGGA
 
CCAAAAACGGCGACUCAUCCGUCCUGAGUGGCCUCUGGUGUGGAAACCAGAGAGUCUUGCGGU
AACCAACGAAAUCUCUUCGGAGUGGGUGUUGGGGACCACCAAAUGAAGAAAAUAGCCCCUCCCU
CGCGCAGGGGGGGGCCAGCCC
   FGF9 (SEQ ID NO. 13): 
UGAAACAGCAGAUUACUUUUAUUUAUGCAUUUAAUGGAUUGAAGAAAAGAACCUUUUUUUUC
UCUCUCUCUCUGCAACUGCAGUAAGGGAGGGGAGUUGGAUAUACCUCGCCUAAUAUCUCCUGG
GUUGACACCAUCAUUAUUGUUUAUUCUUGUGCUCCAAAAGCCGAGUCCUCUG
   HCRSV (SEQ ID NO. 14): 
GUUUGUAUUGUAUGGGCCUCCGUGAAGUUAGGGGCAGUGGAAACGUCGGUCUAGCCAGCCGU
CCCCUGGGUAGUGUGCUCUGUCUAUGUACACCACUACUCGGUUUCCACAAACGAUCGGACUCCC
AAUAUAGUGUACAUUAUGUUGAUUGCUUCUUUCUACCUAUAUAAUAUCUCCUCUCAUCUCUCC
UCUACGCUUGGAAUCCAUAAAAUCCUUCGGGUGGGCUUGGAUUUCGGGUACUUGUUUGAAAU
UCCCUCACGACCGCCAUAGUUGUGAGGCAGCCC
   HIF-α (SEQ ID NO. 15): 
GUGCUGCCUCGUCUGAGGGGACAGGAGGAUCACCCUCUUCGUCGCUUCGGCCAGUGUGUCGGG
CUGGGCCCUGACAAGCCACCUGAGGAGAGGCUCGGAGCCGGGCCCGGACCCCGGCGAUUGCCGC
CCGCUUCUCUCUAGUCUCACGAGGGGUUUCCCGCCUCGCACCCCCACCUCUGGACUUGCCUUUC
CUUCUCUUCUCCGCGUGUGGAGGGAGCCAGCGCUUAGGCCGGAGCGAGCCUGGGGGCCGCCCGC
CGUGAAGACAUCGCGGGGACCGAUUCACC
   P53A (SEQ ID NO. 16): 
UCUAGAGCCACCGUCCAGGGAGCAGGUAGCUGCUGGGCUCCGGGGACACUUUGCGUUCGGGCU
GGGAGCGUGCUUUCCACGACGGUGACACGCUUCCCUGGAUUGGCAGCCAGACUGCCUUCCGGG
UCACUGCC
   RCNMV (SEQ ID NO. 17): 
UUGGUUCUUUUAAGUGUAGCCUCCACCCGAGUUGCAAGAGGGGAACACGCAGUCUCGCCGACCC
UGUUGGCAAACAGUAAAAUUGCAAAAAAUAGAGUGCUAGGAGUAGUUCCCGUACCCGCGGGAG
CAAGACCCUACUACAGUAGACGAACCGGCAUCGGACCCUGGGAAACAGGUACCUAGCGUAUUAA
UAGGUCGCUAGAAAUCGGGCGGGCUUCACCUUCGGGUAACCCGCCGUCGAGAGGGAAAACCUCU
GUGUGGUUGUGCGCACGUCGUUGUCGGAAGACUCUCACUGUUGUAGUUAUUUCCUUUUUCUU
UUAGGUAGGAGCACUUGCUCAGAGGGCGCAUACCUCUGGUAAAACAAAAUUGGCCCCUGAGAG
UAGAAAACUCAGGUUAAUAAAACAGGAGAAUAUUUACCUAGUCGUAUAACGGCUAGGUACCCC
   PCRPV (SEQ ID NO. 18): 
UUAUUUGUAUAUGUUAUGUAUAAUUAUAAUCAUGCAAUGGCACAUGUAGUUCCCCUAACCUCU
CAGUAGGAGUCAAGCCCUGAUGCGCCAACAUCCAGAAGGGGGUAAAGCUGAGUCGGUGCAAGG
AACCUUGUUAGCCCUAAGAAAAACCCAAAAACAGAGUGCAUAGUUUAGCUUUUGUGUUGUAGG
AGCCAGAGACCAAAGAUCCAGACAACUGGUGAGGGCGGUCGAUGGUGCGAAGUAAAUUAGAAA
GGGGGGCGUUUACCCACGUCCUCCCUUGCCC
   PMV (SEQ ID NO. 19): 
ACCUGCCCAAGGUUAUCACAGGCUAGGACCGGCCUGAUAAAGGUCCGACUUGAUGUGCAGCUUC
UUAGGUGCAUCUCGUAUAGAAGCGCUCUGGACAUGACACCUUUGAGACAGACCGUACAGCAGU
CACACGGGACGCCACACCACCUUUGCAGAGGUGCCCUUGGGAAACCAAUGGUGUGGGGUGACAC
UGAUUAGUCGUUAACGGUGUACUAAUCCAGAGUAGUUCAGUGCAGGGGAAACCCGGGCCGUAA
GCACGUGGAACUAUAUAACUUAAUCUCGCUGAGAAGAUUGGGGGGGAUUCAUGAAAUCCCCUC
ACCAGGCCC
   CbMV (SEQ ID NO. 20): 
ACACUAAGAGUUACUGCCACCAAAAGAGGAUAAUCAGACCACUUGUAGUUUGCUGCCACAAACU
GGUUGACAAGUUAAUGCCUCUCUCUUAGUUAGUAAGCCGGAUUCAAGUAGGGAAUGUGGAGG
GAGAUGCCAUGGUGGCAUCAGAGCAGACCUGGAAAUACUCUUCGGAGGGGCGCCAGAACUGCAC
UAAACACUUGUUAUCAGGGGACUGUUGAGGAGUCUCCCCGCCCG
   CIRV (SEQ ID NO. 21): 
GUUCGCUGAAACGAGUGUAAAUCUGGCAUAGCAUACAGGUUACUCUUGUUGGGUUCUAGAUG
UUAUGAUGACGAGUCGGUUCGGGCUCCGCACUAGGUUUGGUCACCUAGGGGAUGGAGAUAUG
GAAAGGGUCUCGUGUGCUGUUAGACGGUCGUAAGACGCGCUUGCAACAUGGGCCUAUGACCGG
AUAAGUCAUAGCAAUACUAGCCAACAUGAAUAGGAUUCCUGUUUACGAAAGUUAGAUGUCACU
UGUGGAAACGGACCCAGACACGGUUGAUCUCACCUUCGGGGGGGCUAUAGAGAUCGCUGGAAG
CACUACCGGACAACCGGAACAUUGCAGAAAUGCAGCCC
   GaMV-PTE (SEQ ID NO. 22): 
AGGAAGAUGCUUGGGGGUGUUCCCCUAAAGUUCAGAAUGGUCUAUCAGGGAGCAGGCACUUU
UCCGGCUUUGGCGAGUCGUCUCCACUUGUAUACGAACAGUGAGGGGAAAAGUUAUCUGAUAGA
AGAAUGAACCAUGGACAAGGACACUCUAAACCACCACUAAGAAGCUAUAGGGAAACCUUGCUGA
GGGCAGUGAGUGGUCGUACCAGAACGAAUAUGGCUUUGCCCCGCCC
   HnRSV (SEQ ID NO. 23): 
ACGGGAUGAGCUUCUGGGGGCUGCCACCCUCGCCUCAUGCGCAUUGCCUGCUCUGGGCGCAUG
UGGAAGCUGAUAUAGUUGAGAGCUUCGUGGAAGGAUGUGGAAACCAAGGGAGGGAGAUAUCC
AGGGAGGAUGUCGGAGUUGUAAGGAUAAAAUACCUAAGCUUCAUCAGGUUAGGUUGGGCUUC
CGAUGCAACAAAACACCAGUAGAUAAAUAGGGGGGGAUCCAGGGGAUCCUUCUCCGCCC
   MNeSV (SEQ ID NO. 24): 
GACAAACUCUCCGCCUAAUGUCUGAUGAUGUGAGGAACGUGGACUGUGAUGUGGUGGUGCGG
UACCAUGGCUGGUCACCAUGGUAAUGCGUAGGGCAACACAGUUCAUUAAGACUCACUGAUGAU
GGCACUAGGCACGGUUCACCCCCAUCCUUCGGGAGGGCUAUAGGGGGUGACCGGGUUACACCAC
CGGAAGACCGGAACAUUGCCUUUGGGCAGCCC
   MNSV (SEQ ID NO. 25): 
AUCUCGCCUUAGGUUUACGGUUACCCGAGCUUCGCCUGAAACGGCGUUCCUCUAAUUCCUUCCU
GGUUCUUUCCCUCCACGGCUCGCGUGUUCUGCGGAACUGCAUCUGCUUCACUUCCUUCUUUUU
CCCUCUGUGGUGGUACGACCAAUGGGUGGCUCCCAUUGACUGCGUAGGACAUGGAAAAAGAUU
GAGUGGAGUGCACGAAGCGUCGCAAGAUCGCUGCCUGACGAGGUCCAGCCAAUCAAAUACCAAA
CAUCUGGUUUGGGGGCGGAUUGGCUGUAUGUCAUGUAAGUUUGAGGGGUUCUAUCGGAGCCC
CCCGCCC
 CCCGCCC
   PLPV (SEQ ID NO. 26): 
GGAAAGCCACUGCGGUGGGGUCAGUCUCCUGGUGCGCCAACACCCAGAAGGGGAGACAACUUCA
GUGGUUCAAAACCCGAUGCUUCGUGUAAGCCUAGAAAAAUCCAAAAACAGAGUGCUACUGCUU
UCAGUGUGCGAGCUUUAUUUGCUCACUUCCUCCGUCGAUAAAAUCCCGAAUUUUCGGGUGGGC
UUCGGCGGACAUCCUUAAUGAAGAACGGGGGGGUAACCCACCCCCCUGAUCUGCCC
   PEMV2 (SEQ ID NO. 27): 
GGCUUCGCUUCCCGCCGGAAGACCGCGGCGGUUCUGUUCCUCCCACAGGAGUACGGCAACAACC
CACCUUGGGAAAGUGGGGACCCCAGCACUAACUCCUUUAACUAGGCGGGCGUGCUGGUUACAG
UAGGAGGGGACAGUGCGCAUCAAAACUGAGCCCCACCACAACUCUCAUCCACAGGGUGGUUGGG
ACGCAGGUGUCGGAGGGAUCGCCAGCCCUCAGGAUAGUGAGCUCCCGCAGAGGGAUAAGCUAU
CUCCCUGCGACGUAGUGGUAGAACACGUGGGAUAGGGGAUGACCUUGUCGACCGGUUAUCGGU
CCCCUGCUCCUUCGAGCUGGCAAGGCGCCCAAAGGUUCUACACUGCUACUAAAGUUGGUGGUG
GAUGUCUCGCCCAAAAAGAUCAUAAACGCGCGGGAUAAGGUCCCUUUCCACCUUCGCCGGGUAA
GGCUAGAGUCAGCGCUGCAUGACUAUAACUUGCGGCCGAUCCAGUUGCACGACUGGUGGUCCC
CCUCAGUGUCUUGGUUGUCUGCCAAGUGGGCGGUGGUCGGAUUCCACCACACCCUGCCACGAG
 
GUGCGUGGAGACUUGGCCAGUCUAGGCUCGUCGUAAUUAGUUGCAGCGACGUUAAUCAACCCG
UCCGGGCAUACAAUAGGACCGGUUGUGCUUCUUCCUCCCUUUCUUAGCCAGGGUGGUUACCUC
CCUGGCGCCC
   PFBV (SEQ ID NO. 28): 
GAGGGCAGUCACCCUACCUUCUGCCAGAGGUUCCCGGAGGAGUAGUGAUCCUUUGGGGGUGAU
UCAUAGCUAGGAAAGGUCGGAGAGUUGGGAGGAGGCCAACUGGGGGAGGUGCUAGGAGCACCA
GAUGCAGCAGAGCAAAAAUCCGGGUUUCAUCGCCUGGUGCGGGCGCUCAAUGCUGCUAACACG
UUCCUGAAGGAAGAAAGGUUUCGGUCGAUGGAGACCUUAACCCGCCC
   PSNV (SEQ ID NO. 29): 
CAAUAUGCACGAGCUGUCUUGUAACACAGCAAUGGAGUAAGUGUACUCUGGUAGCAAUGGGUG
GGGUCGAUUUCCUACGUCUAGCUUUGGCCGGCUAGCGUCUCUCGUAGUGGGGAAAUAACUCAU
UGAGACCAGCUCAGAACGCUGACAUUGUCUGUGUUUGGAUAGAGGGCUUGGGGAAAACUCCGU
AAGGAGGGGCGCCCGAGAGCCUAGUAACACCAAAUUUAUAAACUAGGGGGGACCGUUAAGGUC
UCCCCCGCCCC
   TBSV-YSS (SEQ ID NO. 30): 
GUUUGUGGAGAUGAGUGUAAAUCUGGCAUAGCAUACAGGUUACUCUUGUUGGGUUCUGGAUG
UUAGGAUGACGAGUCGACUCGGGCUCCGCACUAGGUUUGGUCGCCUAGGGGAUGGAGAUAUG
GAAAGGGUCUCGUGUGGUAUCAGUCGGUCGAAAGACGCGCUUCCAACAUGGGCCUAUGGUCGG
AUAAGUCUUAGCAAUACCAGCCAGCAUGAAUUGGAUUCCUGUUUACGAAAGUUAGGUGUCACU
UGUGGAAGCGGACCCAGACACGGUUGAUCUCACCCUUCGGGGGGCUAUAGAGAUCGCUGGAAG
CACUACCGGACAACCGGAACAUUGCAGAAAUGCAGCCC
   TCV-TSS (SEQ ID NO. 31): 
UACGGUAAUAGUGUAGUCUUCUCAUCUUAGUAGUUAGCUCUCUCUUAUAUUAAGAAAAGAAAA
CAAAAACCCCCAGGUCGCUUUAUUUUGACCUGUGUUAGGGACCAAAAACGGUGGCAGCACUGUC
UAGCUGCGGGCAUUAGCCUGGAAAACUAGUGCUCUCUGGGUAACCACUAAAAUCCCGAAAGGG
UGGGCUUUGGGGACCCUCCGAACCAAUAGAUAGCCUCCCUCCUCGGACGGGGGGCCUGCCC
   TNV-D (SEQ ID NO. 32): 
UUGCUUUCAUAGACCCGUCUUCCGGGAGACGUUAAGAAGAUGCCCGUGGAAAAUAGUAGGUAG
AGUUGUGGCGUGACAACCCACAACUGCAUCUCUUUGCUUAUCUAGUUACAAUAUAUGUUGACG
UACAAGAAGGAUCCUGGGAAACAGGUUCAACGGGCUCACUGUGGUGGUGAGCCGUCGCAUCAC
 
UUGUAUGUGCUCCAAUAUUGGUUGUCGAUAAGCGUCCUGACAUGGCUCCAUGCAACAGCAUGG
GGGGGUCCAGAGUCAGUCCCCUCUUUAUUUACCUAGAGAUUCCUCUAGGAAUU
 STNV (SEQ ID NO. 33): 
ACCAUGGUCAUGAUGAUGAUAGUGAAGGACGCUGAAAGAUGCGUAGCUACCCUCCUGGUGCAC
UUCCUGGUGCAAAGCAGAACCAAAGGGUACGGUGGUACGGCGGACAGUAGUCCUGAA
   P53BN (SEQ ID NO. 34): 
AUGGAGGAGCCGCAGUCAGAUCCUAGCGUCGAGCCCCCUCUGAGUCAGGAAACAUUUUCAGACC
UAUGGAAACUACUUCCUGAAAACAACGUUCUGUCCCCCUUGCCGUCCCAAGCA
   SCV (PTE-TSS) (SEQ ID NO. 35): 
GUUGCUCGACUGUGAGGGGACCUACCCACUGUGCUGCCACACAGGAACUUCCAACCUUCGGGUG
GCGAGGUAGGGCAGAAGAGUGACUGAACGAGGAUGGUGUAGAGCAAUGGAUGGGGGGGGAGU
CCAUGGUGGGCUCCGGCGCGACGAUGCUAAAAUCUCCAUAUCAUGUGUGGAGUGGGCUGCUUA
GUCGCUUAUUAGUUGAAAAUUAGGGGGGUCCUUAGGGACCUCUCCCGCCC
   SCV (PTE) (SEQ ID NO. 36): 
GUUGCUCGACUGUGAGGGGACCUACCCACUGUGCUGCCACACAGGAACUUCCAACCUUCGGGUG
GCGAGGUAGGGCAGAAGAGUGAC
   PEMV2 (PTE) (SEQ ID NO. 37): 
GGUAGAACACGUGGGAUAGGGGAUGACCUUGUCGACCGGUUAUCGGUCCCCUGCUCCUUCGAG
CUGGCAAGGCGCCCAAAGGUUCUAC
   TPAV (SEQ ID NO. 38): 
ACUCGCUCAGAGUCAUCCUAGGCUAGGACCGGCCAGACAAAGGUCCGACACUACAUGCAGCGCU
CUAGGUGUGUAGUGACAGUAAGCGCGCUCUAUCCGAAACUCCAGUGACAGGACAAGACCCCAGG
CGGGACGCCACACCCAUGUUUGCAGACAUGGCCCGAGGAAACUCUGGUGUGGGCUGGGGGGAA
UCCUAUGAUCACUGGACGGAAGGUAGAGAGUUCCAGCACAACCCCUUUAGGGUGGGCCGUAAG
UGCGUGGGAACAUUUCCUACAGUCUCACUGAGAAGAUUGGACGGGGUACCAUGAGUACCCCUC
ACCAGGCCC
(3) External homologous arm sequences involved in structure optimization:
   20 bp 5' external homologous arm (SEQ ID NO. 39):
      CCGTCGATTGTCCACTGGTC
   20 bp 3' external homologous arm (SEQ ID NO. 40):
      GACCAGTGGACAATCGACGG
   35 bp 5' external homologous arm (SEQ ID NO. 41):
      GCACTGGCTGACAGACCGTCGATTGTCCACTGGTC
   35 bp 3' external homologous arm (SEQ ID NO. 42):
      GACCAGTGGACAATCGACGGTCTGTCAGCCAGTGC
   70 bp 5' external homologous arm (SEQ ID NO. 43): 
CGTACTGCTAGCTCGCTGCCATCGATGCTGATGAGGCACTGGCTGACAGACCGTCGATTGTCCACTGG
TC
   70 bp 3' external homologous arm (SEQ ID NO. 44): 
GACCAGTGGACAATCGACGGTCTGTCAGCCAGTGCCTCATCAGCATCGATGGCAGCGAGCTAGCAGT
ACG
   105 bp 5' external homologous arm (SEQ ID NO. 45): 
CGAATGCGATGCTAGTCGTAGCTAGTCCGATGATGCGTACTGCTAGCTCGCTGCCATCGATGCTGATG
AGGCACTGGCTGACAGACCGTCGATTGTCCACTGGTC
   105 bp 3' external homologous arm (SEQ ID NO. 46): 
GACCAGTGGACAATCGACGGTCTGTCAGCCAGTGCCTCATCAGCATCGATGGCAGCGAGCTAGCAGT
ACGCATCATCGGACTAGCTACGACTAGCATCGCATTCG
   140 bp 5' external homologous arm (SEQ ID NO. 47): 
CGCTGATGCTGATCGTAGCTGGATGCTAGTCGTAGCGAATGCGATGCTAGTCGTAGCTAGTCCGATG
ATGCGTACTGCTAGCTCGCTGCCATCGATGCTGATGAGGCACTGGCTGACAGACCGTCGATTGTCCAC
TGGTC
   140 bp 3' external homologous arm (SEQ ID NO. 48): 
GACCAGTGGACAATCGACGGTCTGTCAGCCAGTGCCTCATCAGCATCGATGGCAGCGAGCTAGCAGT
ACGCATCATCGGACTAGCTACGACTAGCATCGCATTCGCTACGACTAGCATCCAGCTACGATCAGCAT
CAGCG
(4) Internal homologous arm sequences involved in structure optimization:
   10 bp 5' internal homologous arm (SEQ ID NO. 49):
      TCCACTGGTC
   10 bp 3' internal homologous arm (SEQ ID NO. 50):
      GACCAGTGGA
   20 bp 5' internal homologous arm (SEQ ID NO. 51):
      CCGTCGATTGTCCACTGGTC
   20 bp 3' internal homologous arm (SEQ ID NO. 52):
      GACCAGTGGACAATCGACGG
   35 bp 5' internal homologous arm (SEQ ID NO. 53):
      GCACTGGCTGACAGACCGTCGATTGTCCACTGGTC
   35 bp 3' internal homologous arm (SEQ ID NO. 54):
      GACCAGTGGACAATCGACGGTCTGTCAGCCAGTGC
   70 bp 5' internal homologous arm (SEQ ID NO. 55): 
CGTACTGCTAGCTCGCTGCCATCGATGCTGATGAGGCACTGGCTGACAGACCGTCGATTGTCCACTGG
TC
   70 bp 3' internal homologous arm (SEQ ID NO. 56): 
GACCAGTGGACAATCGACGGTCTGTCAGCCAGTGCCTCATCAGCATCGATGGCAGCGAGCTAGCAGT
ACG
   100 bp 5' internal homologous arm (SEQ ID NO. 57): 
aatgataccgtaggagctcatgcggatataCGTACTGCTAGCTCGCTGCCATCGATGCTGATGAGGCACTGGCT
GACAGACCGTCGATTGTCCACTGGTC
   100 bp 3' internal homologous arm (SEQ ID NO. 58): 
GACCAGTGGACAATCGACGGTCTGTCAGCCAGTGCCTCATCAGCATCGATGGCAGCGAGCTAGCAGT
ACGtatatccgcatgagctcctacggtatcatt
   150 bp 5' internal homologous arm (SEQ ID NO. 59): 
agccaagcagtattcatcctgtacacaattccgataactatccctgatggaatgataccgtaggagctcatgcggatataCGTACT
GCTAGCTCGCTGCCATCGATGCTGATGAGGCACTGGCTGACAGACCGTCGATTGTCCACTGGTC
   150 bp 3' internal homologous arm (SEQ ID NO. 60): 
GACCAGTGGACAATCGACGGTCTGTCAGCCAGTGCCTCATCAGCATCGATGGCAGCGAGCTAGCAGT
ACGtatatccgcatgagctcctacggtatcattccatcagggatagttatcggaattgtgtacaggatgaatactgcttggct
   200 bp 5' internal homologous arm (SEQ ID NO. 61): 
aggacgactctggagtacatcttaggcggagaaccgtgatacacaatggaagccaagcagtattcatcctgtacacaattccgataa
ctatccctgatggaatgataccgtaggagctcatgcggatataCGTACTGCTAGCTCGCTGCCATCGATGCTGATGAG
GCACTGGCTGACAGACCGTCGATTGTCCACTGGTC
   200 bp 3' internal homologous arm (SEQ ID NO. 62):

GACCAGTGGACAATCGACGGTCTGTCAGCCAGTGCCTCATCAGCATCGATGGCAGCGAGCTAGCAGT
ACGtatatccgcatgagctcctacggtatcattccatcagggatagttatcggaattgtgtacaggatgaatactgcttggcttccatt
gtgtatcacggttctccgcctaagatgtactccagagtcgtcct

### Example 1. Design of Circular RNA Construct

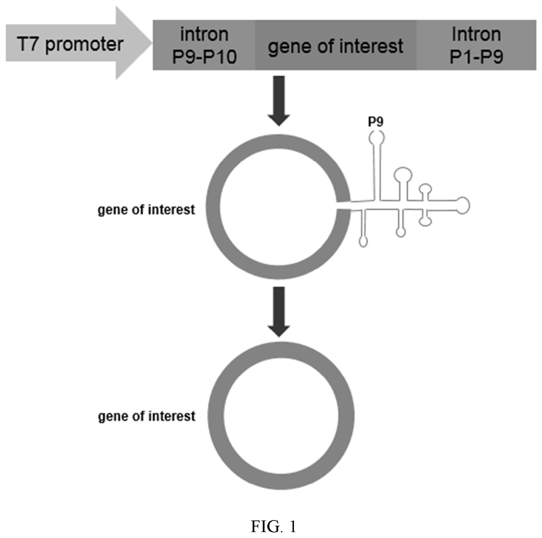

Design and construction of PICC-circRNA structure: the structure comprised a T7 promoter (SEQ ID NO. 1), a 3' intron (SEQ ID NO. 2, i.e., P9-P10), a GOI (sequentially comprising, from the 5' end to the 3' end, the kozak sequence set forth in SEQ ID NO. 3, the IRES sequence set forth in SEQ ID NO. 4, and the EGFP sequence set forth in SEQ ID NO. 5), and a 5' intron (SEQ ID NO. 6, i.e., P1-P9). The schematic diagram of the construction is shown in FIG. 1.

The secondary structure of the group I intron exhibited a high degree of conservation. In the present disclosure, a group I intron derived from *Tetrahymena* was selected, which comprised several characteristic paired regions. These paired regions formed different helical structures according to the principle of complementary base pairing, and these structures were designated as P1-P10 from the 5' end to the 3' end. Meanwhile, different large helical structures were further divided into several small helical subregions. For example, P2 comprised the P2 and P2.1 regions shown in the figure, and P5 comprised the P5, P5a, and P5b regions shown in the figure, and so forth. The 3' intron and the 5' intron described in the present disclosure are two fragments generated by splitting the group I intron in the non-complementary region of P9, where the fragment containing the 3' splice site is the 3' intron, and the fragment containing the 5' splice site is the 5' intron (the schematic structural diagrams are shown in FIGS. 1-3).

### Example 2. Preparation and Preliminary Identification of Circular RNA

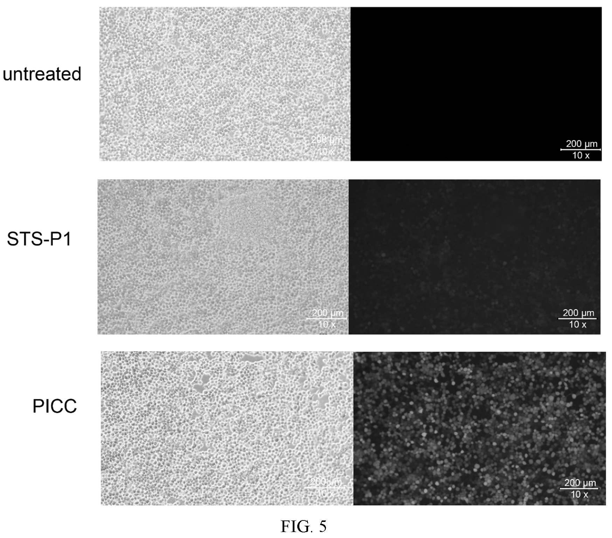

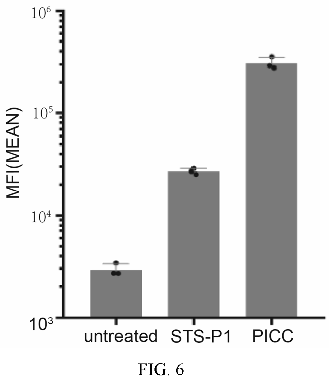

(1) The plasmid encoding GFP constructed in Example 1 was linearized, and a circular RNA was obtained after *in vitro* transcription (IVT), DNase I treatment, and purification. The circular RNA was subjected to reverse transcription-PCR (RT-PCR), and the cDNA product was further identified by PCR to confirm that the product was circular RNA. The target band was sequenced, and the results showed that the circular RNA was successfully prepared and that the ligation site was correct (the results are shown in FIG. 4).
   The correct circular RNA^{GFP} after verification was transfected into HEK293T cells, and GFP expression was observed under a fluorescence microscope after 24 h. The results showed that circular RNA formed by the PICC-P9 splitting method exhibited higher GFP expression than circular RNA formed by the P1 splitting method (the results are shown in FIG. 5), and flow cytometry results further confirmed this result (the results are shown in FIG. 6). PICC represented splitting of the non-complementary regionof P9 in Example 1, STS-P1 represented splitting of the P1 region, and the specific splitting sites were bases at positions 6 and 7 of the sequence set forth in SEQ ID NO. 7.
(2) In the present disclosure, common translation modes (FIG. 7A) were analyzed, and IRES and CITE sequences were compared with respect to sequence length and number of stop codons. It was found that IRES sequences were generally excessively long and contained relatively large numbers of stop codons (FIG. 7B), while CITE sequences were generally shorter and contained fewer stop codons (FIG. 7C). Translation efficiency of the CITE sequence could be improved by adding a stem-loop structure downstream thereof (FIG. 7D). Accordingly, translation abilities of CITE sequences were screened in the present disclosure (FIG. 7E). In particular, the present disclosure found that CITE sequences were compatible with different base modifications (FIG. 7F). Moreover, translation could also be achieved in A549 cells (FIG. 7G), and immunogenicity was reduced (FIG. 7H) and cell viability was improved (FIG. 7I). Therefore, BBV was used to construct PICC, that is, the optimized PICC-circRNA structure comprised a T7 promoter (SEQ ID NO. 1), a 3' intron (SEQ ID NO. 2, i.e., P9-P10), a GOI (sequentially comprising, from the 5' end to the 3' end, the kozak sequence set forth in SEQ ID NO. 3, the BBV sequence set forth in SEQ ID NO. 9, and the EGFP sequence set forth in SEQ ID NO. 5), and a 5' intron (SEQ ID NO. 6, i.e., P1-P9).

Certainly, the present disclosure provides a universal method for preparing circular RNA. Group I introns derived from other species, such as *Anabaena* and bacteriophage T4, can also achieve the same effect. Specifically, selection and splitting of the non-complementary region of P9 of a group I intron, followed by insertion of an RNA sequence to be circularized, can achieve highly efficient circularization. Differences between the "scarless" circular RNA generated by the present disclosure and other group I intron-based circularization schemes are shown in FIG. 8A.

### Example 3. Verification of Circularization of Circular RNA of the Present Disclosure and Improvement of Circularization Efficiency

The plasmid encoding GFP constructed in Example 1 was linearized, and *in vitro* transcription (IVT) was performed for 0.5 h, 2 h, 4 h, and 16 h, respectively. A circular RNA was obtained after DNase I treatment and purification. The results showed that the circularization scheme of the present disclosure exhibited efficient splicing and circularization efficiency (the results are shown in FIG. 8B). Subsequently, the in vitro transcription was performed for 16 h.

In particular, after 16 h of IVT, the product was subjected to GTP treatment to demonstrate that the present disclosure could achieve relatively high circularization efficiency without additional GTP treatment (the results are shown in FIG. 8C). As can be seen from the figure, the circularization efficiency of the PICC group was significantly higher than that of the STS-P1 group and was 27.5 times that of the STS-P1 group, which might be due to the unique splitting mode of the present disclosure greatly improving the circularization efficiency.

The purified circular RNA product was further subjected to RNase R treatment, and the circular RNA before and after treatment was identified by urea-PAGE gel analysis. It was found that the circular RNA bands were significantly enriched, and the linear RNA bands were degraded (the results are shown in FIG. 8D). Further, the circular RNA was subjected to reverse transcription-PCR (RT-PCR), and the cDNA product was further identified by PCR to confirm that the product was circular RNA. The target band was sequenced, and the results showed that the circular RNA was successfully prepared and that the ligation site was correct (the results are shown in FIG. 8E).

### Example 4. Addition of Homologous Arms to Improve Circularization Efficiency

### (1) Addition of external homologous arms

Design and construction of PICC-circRNA structure with addition of external homologous arms of different lengths: the structure comprised a T7 promoter, a 5' external homologous arm, a 3' intron, a GOI, a 5' intron, and a 3' external homologous arm. "Different lengths" referred to 20 bp, 35 bp, 70 bp, 105 bp, and 140 bp 5' external homologous arms and 3' external homologous arms (SEQ ID NOs. 39-48).

The above plasmids encoding GFP and containing homologous arms of different lengths were linearized, and a circular RNA was obtained after *in vitro* transcription (IVT), DNase I treatment, and purification. The circularization efficiency of the obtained circular RNA was determined by urea-PAGE gel analysis (the results are shown in FIG. 8F). It could be seen that addition of homologous arms of different lengths slightly improved the circularization efficiency compared with the case without homologous arms.

### (2) Addition of internal homologous arms

Design and construction of circRNA-PICC structure with addition of internal homologous arms of different lengths: the structure comprised a T7 promoter, a 3' intron, a 5' internal homologous arm, a GOI, a 3' internal homologous arm, and a 5' intron. "Different lengths" referred to 10 bp, 20 bp, 35 bp, 70 bp, 100 bp, 150 bp, and 200 bp 5' internal homologous arms and 3' internal homologous arms (SEQ ID NOs. 49-62).

The above plasmids encoding GFP and containing homologous arms of different lengths were linearized, and a circular RNA was obtained after *in vitro* transcription (IVT), DNase I treatment, and purification. The circularization efficiency of the obtained circular RNA was determined by urea-PAGE gel analysis (the results are shown in FIG. 8G). It could be seen that homologous arms of different lengths affected the circularization efficiency, but the circularization efficiency was improved compared with the case without homologous arms. In particular, the circularization scheme used in the present disclosure exhibited efficient circularization efficiency without addition of homologous arms, while the PIE method did not (the results are shown in FIG. 8H).

### Example 5. Circularization Can Be Achieved in the Absence of P10 Structure in Group I Intron

Design and construction of PICC-circRNA structure: the structure comprised a T7 promoter, a 3' intron, a GOI, and a 5' intron. The 3' intron did not comprise the P10 structure of the group I intron used (that is, the structure was generated by splitting the sequence set forth in SEQ ID NO. 8, and the splitting site and other conditions were the same as those in Example 1). The above plasmid encoding GFP was linearized, and a circular RNA was obtained after *in vitro* transcription (IVT), DNase I treatment, and purification. The circular RNA was identified by urea-PAGE gel analysis, and the results showed that circularization could still be achieved in the absence of the P10 structure in the group I intron (The results are shown in FIG. 8I. Although the circularization efficiency was 68.4% of the original efficiency, the design could completely avoid retention of group I intron sequences and would not introduce any exogenous sequence into the prepared circular RNA, thereby further reducing potential effects of residual group I intron sequences on the structure of the circular RNA and reducing immunogenicity of the circular RNA).

### Example 6. Universality of the Present Disclosure for Circularization of Different Genes

Plasmids encoding GFP, human erythropoietin (hEPO), luciferase (Gaussia luciferase), and Cre-recombinase (Cre) were linearized, and a circular RNA was obtained after *in vitro* transcription (IVT), DNase I treatment, and purification. The circular RNA was enriched by RNase R treatment, and the circular RNA was identified by urea-PAGE gel analysis. The results showed that the present disclosure could efficiently circularize RNA of different lengths and exhibited universality (the results are shown in FIG. 8J).

The circRNA encoding GFP was transfected into 293T cells, and protein expression levels of different genes were detected after 24 hours. The results showed that the PICC constructs could efficiently express different proteins (the results are shown in FIG. 8K).

The PICC constructs used above were all constructed in Example 1.

### Example 7. Circular RNA of the Present Disclosure Exhibits Low Immunogenicity

CircRNA comprising CVB3 IRES and Gaussia luciferase sequences, as well as circRNA circularized from POLR2A, were transfected into A549 cells. After 6 h, cellular RNA was extracted and reverse transcribed to obtain cDNA, and expression levels of immunogenicity-related cytokines were determined by quantitative fluorescence PCR. The results showed that the immunogenic response induced by the circular RNA generated by the present disclosure in A549 cells exhibited sequence correlation (the results are shown in FIG. 8L).

### Example 8. Verification of Rolling Circle Translation Based on the Circularization Scheme of the Present Disclosure

The coding sequence in the circular RNA construct of Example 2 (2) was replaced with 3×FLAG-P2A, and translation was initiated using the BBV sequence to encode an ORF without a stop codon. P2A was used to enable protein polymers generated by rolling circle translation to be separated into functional protein monomers. Circular RNA having a 3×FLAG ORF was transfected into 293T cells, and immunoblot analysis was performed on cell lysates after 24 h. Among the 5 types of circular RNAs evaluated, only circRNA-BBV-3×FLAG-P2A was capable of performing rolling circle translation (the results are shown in FIG. 8M). The coding sequence in the circular RNA construct of Example 1 was replaced with the OVA₂₅₇₋₂₆₄ (SIINFEKL) antigen to prepare circular RNA, and sequence screening was performed (the results are shown in FIGS. 8N and 8O). Similarly, HER2 molecules were screened, and it was found that only sequences using 3×FLAG exhibited expression of the relevant protein (the results are shown in FIG. 8P). One interesting phenomenon was that the present disclosure found that linear mRNA appeared to have relatively weak ability for translating short peptides; and after linear mRNA encoding 3×FLAG formed in vitro was transfected into 293T cells, expression of the target protein was not detected by western blot.

### Example 9. Preparation of Circular RNA Vaccine

The coding sequence in the circular RNA construct of Example 1 was replaced with an RBD antigen to prepare a circular RNA vaccine. First, 293T cells were transfected, and antigen expression was verified by WB. Subsequently, BALB/c-nu/nu mice were injected through an LNP delivery system (15 µg/mouse), and administration was performed once at week 0 and once at week 2. After week 4, innate immunogenicity (comprising MCP-1, IL-6, IP-10, TNF-α, IFN-α, and RANTES), humoral immune levels (comprising binding antibodies and neutralizing antibodies), cellular immune levels (related cytokine detection such as IL-4, IL-6, IFN-γ, TNF-α, and IL-2), and the ability to prevent infection in a challenge model were evaluated. The results showed that the vaccine preparation was efficient, exhibited high humoral and cellular immune levels, and had strong infection prevention ability.

### Example 10. Preparation of Circular RNA Tumor Vaccine

The circular RNA construct capable of efficiently encoding the OVA₂₅₇₋₂₆₄ (SIINFEKL) antigen obtained after screening in Example 8 was prepared as a circular RNA vaccine. The vaccine was injected through an LNP delivery system into immunocompetent BALB/c mice bearing CT26 subcutaneous colorectal carcinoma expressing chicken ovalbumin antigen (OVA) (the results are shown in FIG. 9A). When the CT26-OVA tumor volume reached about 60 mm³, the mice were randomly grouped and immunized by intramuscular injection (i.m.) with PBS, 12 µg of LNP-encapsulated in vitro-transcribed mRNA encoding full-length OVA antigen (mRNA-OVA_{FL}), 12 µg of LNP-encapsulated in vitro-transcribed mRNA encoding the OVA₂₅₇₋₂₆₄ antigen (mRNA-OVA₂₅₇₋₂₆₄), 12 µg of LNP-encapsulated circRNA encoding the OVA₂₅₇₋₂₆₄ peptide generated by the PIE strategy (PIE-circRNA-OVA₂₅₇₋₂₆₄), or 12 µg of LNP-encapsulated PICC-circRNA-OVA₂₅₇₋₂₆₄, respectively, three times at intervals of 3 days. Tumor volumes of the treated mice were then monitored (the results are shown in FIG. 9A). The results showed that, compared with the PBS group, the PICC-OVA₂₅₇₋₂₆₄ group significantly inhibited CT26 tumor growth and was significantly superior to the other three RNA vaccine groups, comprising PIE-circRNA-OVA₂₅₇₋₂₆₄, mRNA-OVA₂₅₇₋₂₆₄, and mRNA-OVA_{FL} groups (FIG. 9B). The PICC-circRNA-OVA₂₅₇₋₂₆₄ group also significantly prolonged survival time of the tumor-bearing mice compared with the PBS, PIE-circRNA-OVA₂₅₇₋₂₆₄, mRNA-OVA₂₅₇₋₂₆₄, and mRNA-OVA_{FL} groups (FIG. 9C), indicating that rolling circle translation-compatible PICC-circRNA has potential as a novel cancer vaccine.

Next, the present disclosure compared PICC-circRNA with N1mΨ-modified mRNA (N1mΨ-mRNA) (the results are shown in FIG. 9D). Similarly, BALB/c mice bearing subcutaneous CT26-OVA colorectal carcinoma were intramuscularly injected with PBS, 15 µg of LNP-encapsulated N1mΨ-mRNA-OVA_{FL}, or 15 µg of LNP-encapsulated PICC-circRNA-OVA₂₅₇₋₂₆₄, and tumor volumes of the treated mice were monitored (the results are shown in FIG. 8D). The results showed that, compared with the PBS or N1mΨ-mRNA-OVA_{FL} group, the PICC-circRNA-OVA₂₅₇₋₂₆₄ group effectively inhibited tumor growth and prolonged survival time of the tumor-bearing mice (the results are shown in FIGS. 9E and 9F). At the end of the experiment, tumor tissues from mice injected with the PICC-circRNA-OVA₂₅₇₋₂₆₄ or N1mΨ-mRNA-OVA_{FL} vaccine were collected, and infiltration of immune cells was analyzed by flow cytometry. The results showed that the PICC-circRNA-OVA₂₅₇₋₂₆₄ vaccine promoted infiltration of CD45⁺ immune cells into the tumor microenvironment, where the proportion of CD8⁺ T cells significantly increased, while the proportion of immunosuppressive Treg cells decreased. In addition, it was also found that immunization with the PICC-circRNA-OVA₂₅₇₋₂₆₄ vaccine increased the proportion of pro-inflammatory cells, indicating that the PICC-circRNA-OVA₂₅₇₋₂₆₄ vaccine could remodel the tumor microenvironment in the mouse model into a pro-inflammatory state (FIG. 9G).

Furthermore, tumor tissue samples from treated mice were collected for hematoxylin-eosin (H&E) staining and immunohistochemistry (IHC) staining. H&E staining results showed that the PICC-circRNA-OVA₂₅₇₋₂₆₄ treatment group exhibited significantly increased tumor cell death, while the PBS or N1mΨ-mRNA-OVA_{FL} group showed diffuse growth and dense arrangement of tumor cells in CT26-OVA colorectal carcinoma-bearing mice (FIG. 9H). IHC staining results showed that infiltration of CD8⁺ T cells in CT26-OVA colorectal carcinoma-bearing mice in the PICC-circRNA-OVA₂₅₇₋₂₆₄ group was significantly higher than that of the PBS or N1mΨ-mRNA-OVA_{FL} group (FIG. 9I).

### Example 11. Preparation of circular RNA CAR-M/T

The coding sequence in the circRNA construct of Example 1 was replaced with a HER2-CAR molecule to prepare circular RNA. First, circRNA^{HER2-CAR} was transfected into Jurkat, THP-1, and J774A.1 cells. The prepared CAR-T/M cells were then co-incubated with SK-OV-3-LUC and MC38-HER2-LUC cells at different effector-to-target ratios (effector cells:tumor cells = E:T, 0.25:1, 1:1, 4:1, and 16:1), respectively, and killing effects of the CAR-T/M cells on tumor cells were detected by luciferase assays. The circRNA^{HER2-CAR} was injected into C57BL/6 subcutaneous tumor-bearing mice (SK-OV-3-LUC and MC38-LUC-HER2 cells, 1 × 10⁶ cells/100 µL) through a targeted delivery system. When the tumor volume reached 100 mm³, LNP-circRNA^{HER2-CAR} (15 µg/mouse) was administered by intravenous injection and multi-point intratumoral injection once every three days for a total of three administrations. At the experimental endpoint, tumors, lymph nodes, spleens, and peripheral blood were collected, cells were isolated, and proportions of T cells and M1 macrophages, as well as expression of circRNA^{HER2-CAR} therein, were detected by flow cytometry. Survival status and tumor volumes of the mice were observed and recorded daily. The results showed that in vivo in situ preparation of circRNA^{HER2-CAR} was efficient and exhibited strong anti-tumor ability.

### Example 12 Preparation of Circular RNA Therapeutic Drug for Autoimmune Diseases

The coding sequence in the circular RNA construct of Example 2 (2) was replaced with an amino acid coding sequence of MOG₃₅₋₅₅, and 70 bp external homologous arms together with 12 bp internal homologous arms were added to prepare a circular RNA vaccine, followed by sequence screening. First, 293T cells were transfected, and antigen expression was verified by WB. Subsequently, C57BL/6 mice were injected through an LNP delivery system (20 µg/mouse) for treatment of EAE disease. After induction of EAE disease in the mice, administration was performed once on day 7 and day 10, respectively, or when the EAE score reached 1-2. Disease severity after administration was evaluated in the mice when disease progression reached scores between 1 and 2. Frequencies of CD4⁺ T cells and MOG₃₅₋₅₅-specific CD4⁺ T cells in spinal cords of mice treated on day 7 and day 10 after disease induction were determined (n = 3-4). One day before EAE induction, Thy1.1⁺ 2D2 CD4⁺ T cells were transferred into Thy1.2⁺ recipient mice, and target organs were analyzed on day 16 after disease induction. On day 16 after EAE induction, CD4 staining of mouse spinal cords was analyzed, and IFN-γ and IL-17a factors secreted in the brain and spinal cord were analyzed. The results showed that treatment with antigen-encoding circular RNA capable of performing rolling circle translation could improve EAE disease in mice.

It is obvious that the examples described above are merely provided for clear illustration and are not intended to limit the embodiments. Changes or modifications in other forms may be made by those of ordinary skill in the art based on the description described above. It is unnecessary and impossible to exhaust all the embodiments herein. Obvious changes or modifications derived therefrom still fall within the scope of protection of the present disclosure.

## Claims

1. A ribonucleic acid construct, comprising, from a 5' end to a 3' end, elements of:
a 3' intron portion comprising a 3' splice site;
a gene of interest (GOI) sequence; and
a 5' intron portion comprising a 5' splice site,
wherein the 3' intron and the 5' intron are respectively two fragments obtained by splitting a group I intron, the group I intron at least comprises, from the 5' end to the 3' end, 9 paired double-helical regions P1-P9, the splitting site is located in a non-complementary region within the P9 region from the 5' end to the 3' end of the group I intron or in a region adjacent to the non-complementary region within the P9 region, the 3' intron at least comprises a part of the P9 region at the 3' end of the group I intron, and the 5' intron at least comprises regions P1-P8 and a part of the P9 region at the 5' end of the group I intron;
a distance between the region adjacent to the non-complementary region within the P9 region and the 5' end or the 3' end of the non-complementary region of the P9 region is 0-40 nucleotides;
the group I intron is unmodified or has been subjected to optimization modification.

2. The ribonucleic acid construct according to claim 1, wherein the group I intron at least comprises, from the 5' end to the 3' end, 10 paired double-helical regions P1-P10, and the splitting site is located in a non-complementary region within the P9 region from the 5' end to the 3' end of the group I intron or in a region adjacent to the non-complementary region within the P9 region.

3. The ribonucleic acid construct according to claim 1, wherein the group I intron is derived from Tetrahymena, Anabaena, or bacteriophage T4.

4. The ribonucleic acid construct according to claim 1 or 2, wherein the optimization modification of the group I intron at least comprises one of:
(1) modifying any one site in a complementary region of P1-P9 or P1-P10, the modification comprising one or more of addition, deletion, and mutation; and
(2) modifying any one site in a non-complementary region of P1-P9 or P1-P10, the modification comprising one or more of addition, deletion, and mutation.

5. The ribonucleic acid construct according to claim 1, further comprising a sequence for initiating translation.

6. The ribonucleic acid construct according to claim 5, wherein the sequence for initiating translation comprises one or more of an internal ribosome entry site sequence, an m6A motif, a cap-independent translation enhancer sequence, and a kozak sequence.

7. The ribonucleic acid construct according to claim 6, wherein:
(1) the internal ribosome entry site sequence is derived from Simian V4, CVB3, CVB5, CVB1, HRV-A89, HRV-B3, Salivirus A GUT, EV107, CVA3, HRV-B37, EchoV11, HRV-C54, HRV-B4, EV-D94, HRV-B93, EV-J, SwineVesicular, CVA20, PV1, HRV-A1, Salivirus A SZ1, Salivirus FHB, EMCV-cf, HCV, Aichivirus, CrPV, Covid 19, Crohivirus B, EchoV-E11, Sapevirus, or Phopivirus; and / or
(2) the cap-independent translation enhancer sequence is derived from a 5'UTR or a 3'UTR of PMV, CarMV-PTE, CbMV, CCFV, CIRV, GaMV, HCRSV, HnRSV, MNeSV, MNSV, PLPV, PEMV2, PFBV, PSNV, RCNMV1, SCV, TBSV, TCV, TNV, BBV, BYDV, STNV, SCV, PEMV2, TPAV, or STNV virus; or is derived from a 5'UTR or a 3'UTR of a HIF-1α, FGF9, p53A, or p53B gene in the Homo sapiens genome.

8. The ribonucleic acid construct according to claim 1 or 5, further comprising a sequence for promoting circularization and expression.

9. The ribonucleic acid construct according to claim 8, wherein the sequence for promoting circularization and expression comprises one or more of a homologous arm, a spacer sequence, and a UTR sequence.

10. The ribonucleic acid construct according to claim 9, at least comprising one of:
(1) the homologous arm comprising an external homologous arm and/or an internal homologous arm;
(2) the spacer sequence comprising a polyA sequence and/or a polyAC sequence; and
(3) the UTR sequence comprising a 5'UTR sequence and/or a 3'UTR sequence.

11. The ribonucleic acid construct according to claim 10, at least comprising one of :
(1) when the homologous arm is an external homologous arm, in an RNA construct, the external homologous arm is located at the 5' end of the 3' intron and/or at the 3' end of the 5' intron; when the homologous arm is an internal homologous arm, in an RNA construct, the internal homologous arm is located at the 3' end of the 3' intron and/or at the 5' end of the 5' intron;
(2) a length of the homologous arm is not less than 10 bp;
(3) the spacer sequence is located upstream of the sequence for initiating translation and/or downstream of the GOI; and
(4) the 5'UTR sequence is located upstream of the sequence for initiating translation, and the 3'UTR sequence is located downstream of the GOI sequence.

12. The ribonucleic acid construct according to claim 1, wherein a coding sequence of the gene of interest comprises one or more of a viral antigen sequence, a tumor antigen sequence, a monoclonal antibody sequence, a bispecific antibody sequence, a CAR sequence, a cytokine sequence, a coagulation factor sequence, a growth factor sequence, a gene-editing enzyme sequence, a fluorescent protein sequence, and a luciferase sequence.

13. A circular RNA, obtained by circularizing the ribonucleic acid construct according to any one of claims 1-12.

14. A preparation method for a circular RNA, comprising circularizing the ribonucleic acid construct according to any one of claims 1-12 to obtain the circular RNA.

15. The preparation method according to claim 14, wherein the circularization comprises steps of in vitro transcription, DNaseI treatment, and purification.

16. The preparation method according to claim 14, wherein modified nucleotides are added during a preparation process at an addition ratio of 0%-100%.

17. A recombinant cell comprising the ribonucleic acid construct according to any one of claims 1-12, the circular RNA according to claim 13, or the circular RNA obtained by the preparation method according to any one of claims 14-16.

18. A method for producing a protein or polypeptide, comprising: introducing the ribonucleic acid construct according to any one of claims 1-12, the circular RNA according to claim 13, or the circular RNA obtained by the preparation method according to any one of claims 14-16 into a host cell for protein expression, wherein the gene of interest encodes the protein or polypeptide.

19. Use of the ribonucleic acid construct according to any one of claims 1-12, the circular RNA according to claim 13, the circular RNA obtained by the preparation method according to any one of claims 14-16, or the recombinant cell according to claim 17 in a preparation of a diagnostic product, a prophylactic product, or a therapeutic product.

20. A medicament, comprising the ribonucleic acid construct according to any one of claims 1-12, the circular RNA according to claim 13, the circular RNA obtained by the preparation method according to any one of claims 14-16, or the recombinant cell according to claim 17.

21. The medicament according to claim 20, further comprising a pharmaceutically acceptable carrier.

22. The medicament according to claim 20, wherein the medicament is a vaccine; the vaccine comprises the ribonucleic acid construct or the circular RNA, wherein the gene of interest comprises an antigen-encoding sequence.

23. The medicament according to claim 20, wherein the medicament is a CAR immunotherapeutic medicament; the CAR immunotherapeutic medicament comprises the ribonucleic acid construct or the circular RNA, wherein the gene of interest comprises a chimeric antigen receptor-encoding sequence.

24. The medicament according to claim 20, wherein the medicament is a medicament for treating an autoimmune disease; the medicament for treating the autoimmune disease comprises the ribonucleic acid construct or the circular RNA, wherein the gene of interest comprises a sequence encoding an antigen capable of inducing immune tolerance.
